Europäisches Patentamt

European Patent Office

Office européen des brevets

(19)

(11) Publication number: **0 054 078**
**B1**

# EUROPEAN PATENT SPECIFICATION

(45) Date of publication of patent specification: **09.04.86**

(51) Int. Cl.⁴: **A 61 F 11/04**

(21) Application number: **80107803.1**

(22) Date of filing: **11.12.80**

(54) Tactile aid to speech reception.

(43) Date of publication of application:
**23.06.82 Bulletin 82/25**

(45) Publication of the grant of the patent:
**09.04.86 Bulletin 86/15**

(84) Designated Contracting States:
**DE FR GB IT SE**

(56) References cited:
**FR-A-1 309 425**
**FR-A-1 515 175**
**GB-A-1 402 508**

(73) Proprietor: **SCOTT INSTRUMENTS COMPANY**
**815 North Elm Street**
**Denton Texas 76201 (US)**

(72) Inventor: **Scott, Brian Lee**
**1500 Sandy Creek**
**Denton, Denton Cty. Texas 76201 (US)**

(74) Representative: **UEXKÜLL & STOLBERG**
**Patentanwälte**
**Beselerstrasse 4**
**D-2000 Hamburg 52 (DE)**

**Description**

The invention relates to a tactile aid for persons having a hearing impairment including a filter array responsive to an acoustic signal and having a high pass channel, a low pass channel and a midfrequency channel each providing an output frequency, means for modulating the amplitude of a random frequency signal by the output frequency of the high pass channel to generate a high pass modulated signal, means for modulating the amplitude of the random frequency signal by the output of the midfrequency channel to generate a midfrequency modulated signal, and vibrating means for transducing the modulated signals.

The spectrograph is the time-honored tool for study of speech perception for visually displaying segregated frequency patterns in space. Recently, however, there is evidence to show that many differences exist between a speech spectrograph and a neural output of a cochlea. For example, the output of the cochlea is linear up to about 1,000 Hz and logarithmic above that, whereas the spectrograph is either logarithmic or linear. Thus, there is serious question whether the temporal processing of acoustical components of speech by the ear can be demonstrated by a speech spectrogram. This is not considered to be a trivial shortcoming of the speech spectrogram given that the fundamental frequency and the first formant information of speech are coded, at least to some extent, by temporal information.

Although it can now be shown that there is a basic problem with the use of the speech spectrogram for voice analysis, the primary problem appears to be that the exclusive use of the spectrograph for speech analysis confines the concept of the speech signal to a spatio-temporal display. Such a spatio-temporal display is not realized to be quite unlike the signal as it exists in space. An acoustic speech signal does have spatial property, but they are not necessarily related to the spatial separation frequencies manifested in the speech spectrogram. The acoustic speech signal is a frequency-integrated, complex waveform whose actual spatial properties are indicated by radiation and reflection, not by frequency. The acoustic world we live in is not fully represented by a speech spectrogram.

Heretofore, the reliance on the speech spectrograph as the sole source of information about the speech signal has restricted observations to an analog of the neural output of the cochlea. This has resulted in the missing of the hypothesized high order free integration stage of perceptual analysis. It is now understood that a more complete analysis of a speech signal required a study of the speech waveform in addition to the spectrogram. The spectrogram provides the components of the signal (spatial) and the waveform shows how the components are integrated in time and space.

FR—A—1 515 175 discloses a tactile aid for persons having a hearing impairment, said tactile aid comprising a plurality of piezo-electric elements which are activated in response to an analyser circuit. The piezo-electric elements are arranged as a matrix of elements which transfer separated spectral information into spatially arrayed areas on the skin. Such type aids encounter difficulties when ordering spectral information in time, since it has as many separate time envelopes as it has spectral channels.

FR—A—1 309 425 discloses a method and an apparatus for transforming acoustic signals into a plurality of coded discreet frequencies which are applied through a band pass filter to a loudspeaker. The said apparatus is intended to re-educate people with hearing impairment and to reduce acoustical information in the higher frequency range to lower frequency information which will be understood more easily by persons having a hearing impairment.

It is an object of this invention to provide an apparatus for analyzing the speech waveform for kinds of information not readily observable from a spectrogram, which apparatus constitutes a tactile speech reception aid for the deaf.

In accordance with the present invention, information about a speech signal is presented to an observer by a tactile aid including first vibrating means responsive to the high pass modulated signal and the output frequency of the low pass channel to produce a vibrating motion, and second vibrating means responsive to the midfrequency modulated signal and the output frequency of the low pass channel to produce a second vibrating motion.

The tactile aid to speech reception according to the present invention presents information about a speech signal to an observer through the skin by means of vibrator transducers. The tactile aid utilizes the waveform envelope to provide a single, slowly varying reference for the many rapid spectral changes in speech. This provides the perceptual system of the observer with a means of integrating information over relatively long periods of time (across syllables). The importance of this is to maintain the temporal order of speech elements for the observer. The aid of the present invention develops a single time envelope for the integration of spectral information by means of discretely placed vibrators.

The present invention therefore provides a tactile aid to speech reception that retains an integrated amplitude envelope and enables the observer to discriminate sentence length material. Further, in accordance with the present invention, a tactile aid conveys as much spectral information to the skin as possible without sacrificing the waveform envelope.

For a more complete understanding of the present invention and the advantages thereof, reference is now made to the following description taken in conjunction with the accompanying drawings.

Referring to the drawings:

FIGURE 1 is a block diagram of a three channel tactile aid for speech perception in accordance with the present invention; and

FIGURES 2a, 2b and 2c schematically illustrate one embodiment of the present invention for responding to a speech signal perception for persons having a hearing impairment.

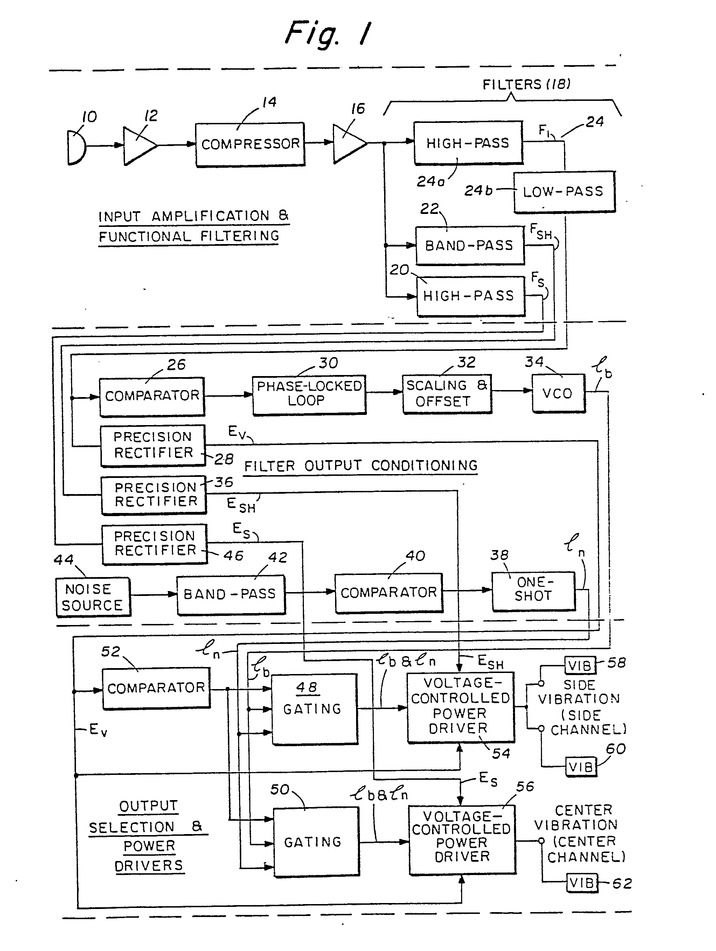

With reference to the figures, the tactile aid Figure 1 receives a speech signal and divides the signal into three spectral channels: (1) a high pass channel, (2) a midfrequency channel, and (3) a low frequency channel. The speech signal is received at a microphone 10 and input to a preamplifier 12 having an output applied to a signal compressor 14 all of conventional construction. A compressed signal output from the signal compressor 14 is applied to a preamplifier 16 having an output interconnected to a filter array 18 that includes a high pass channel 20, a band pass (midfrequency) channel 22 and a low pass channel 24. The low pass channel includes both a high pass filter 24a and a low pass filter 24b. In one embodiment of the invention, the high pass channel 20 operated at a frequency of 8 KHz, the midfrequency channel 22 operated at 2.4 KHz and the low frequency channel 24 operated at from 250 Hz to 900 Hz.

An output signal from the low pass channel 24 at the low pass filter 24b is input to a comparator 26 and to a precision rectifier 28. The output of the precision rectifier 28 is a voltage that varies with the output of the low pass channel 24.

Connected to the output of the comparator 26 is a phase locked loop 30 that provides an output to a scaling and offset network 32 for producing a voltage for driving a voltage controlled oscillator 34. Thus, the low frequency channel serves to detect the first formant of vowel sounds and converts the sounds into a low frequency signal (15 to 200 Hz) at the output of the voltage controlled oscillator 34. These low frequency sounds are detectable when applied to the skin of a person utilizing the tactile aid of the present invention.

An output of the midfrequency channel 22 is input to a precision rectifier 36 that provides a signal for amplitude modulating a random frequency signal.

The random frequency signal is generated at the output of a one shot multivibrator 38 that is driven by the output of a comparator 40 having an input connected to a band pass filter 42 connected to the input of the band pass filter 42 is a random frequency generator (noise source) 44. The output of the one shot multivibrator is the noise source to be amplitude modulated by the output of the precision rectifier 36.

Also amplitude modulating the noise source from the one shot multivibrator 38 is the output of a precision rectifier 46 having an input connected to the high pass channel 20. Thus, the output of the precision rectifier 36 is a midfrequency modulating signal for the noise source from the one shot multivibrator 38 and the output of the precision rectifier 46 is a high pass modulating signal also for amplitude modulating the output of the one shot multivibrator.

To modulate the noise source at the output of the one shot multivibrator 38 this signal is applied to inputs of gating logic 48 and 50. Also input to the gating logic is the output of the voltage controlled oscillator 34 and the output of a comparator 52. The input to the comparator 52 is tied to the output of the precision rectifier 28, which is the low pass channel output, and this signal is connected to voltage controlled power drivers 54 and 56. Also connected to the power driver 54 is the output of the precision rectifier 36 and the output of the gating logic 48. Connected to the power driver 56 is the output of the precision rectifier 46 and the output of the gating logic 50.

By means of the gating logic 48 and the power driver 54 the noise source at the output of the one shot multivibrator 38 is amplitude modulated by the envelope from the midfrequency channel 22 with the output of the power driver 54 connected to vibrators 58 and 60. By means of the power driver 56 and the gating logic 50 the noise source is also amplitude modulated by the output of the high pass channel 20 with the output of the power driver connected to a vibrator 62.

To utilize the tactile aid of Figure 1, the vibrators 58, 60 and 62 are placed on the abdomen of a person just below the rib cage. The vibrators 58 and 60 are placed on opposite sides of the abdomen with the vibrator 62 placed between these vibrators. The center vibrator 62 serves as a transducer for the high frequency channel as well as low frequency vowel sounds by the interconnection of the output of the precision rectifier 28 to the power driver 56. The vibrators 58 and 60 carry both midfrequency speech signals and vowel information. Thus, the low frequency channel serve to detect the first formant of vowel sounds which is fed to all three vibrators by means of the interconnection to the power drivers 54 and 56.

The sensations produced by the vibrators 58, 60 and 62 of the tactile aid of Figure 1 are: (1) periodic, low frequency vibrations spread over a large surface, (2) aperiodic, high frequency stimulation (high frequency for the vibro-tactile-sense) spread over a large surface, and (3) aperiodic, high frequency stimulation over a small surface area (the center vibrator 62).

As an example of the sensations produced by the tactile aid of the present invention, a high frequency fricative, such as [s], is conveyed as a high frequency, aperiodic sensation at the center of the chest of the person utilizing the aid of the present invention. A fricative with lower frequency, such as [sh] is felt as an aperiodic sensation spread over a large area. Vowel sounds are felt as periodic sensations spread over a larger area. Since vowel signals are felt in the center of the chest, the tactile aid of this invention preserves the time integrated envelope of the original speech signal input to the microphone 10. The speech waveform eminates from a single point in space, a speaker, and is perceived through the auditory system as such. A person utilizing the tactile aid of the present invention also perceives the speech sound as coming from a single point by means of sensations produced by the vibrators 58, 60 and 62 mounted as explained.

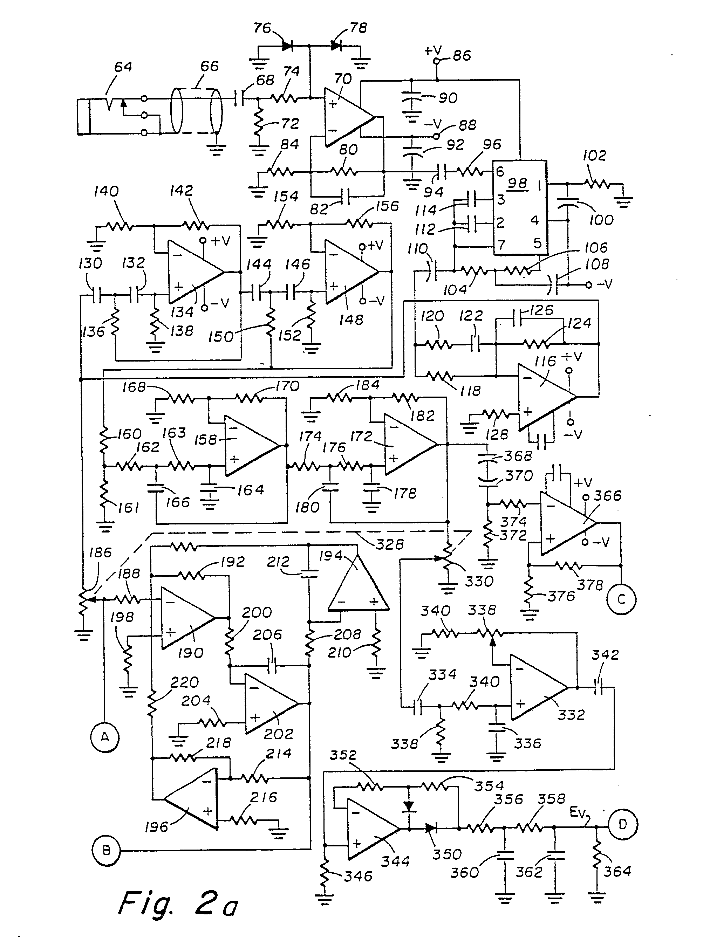

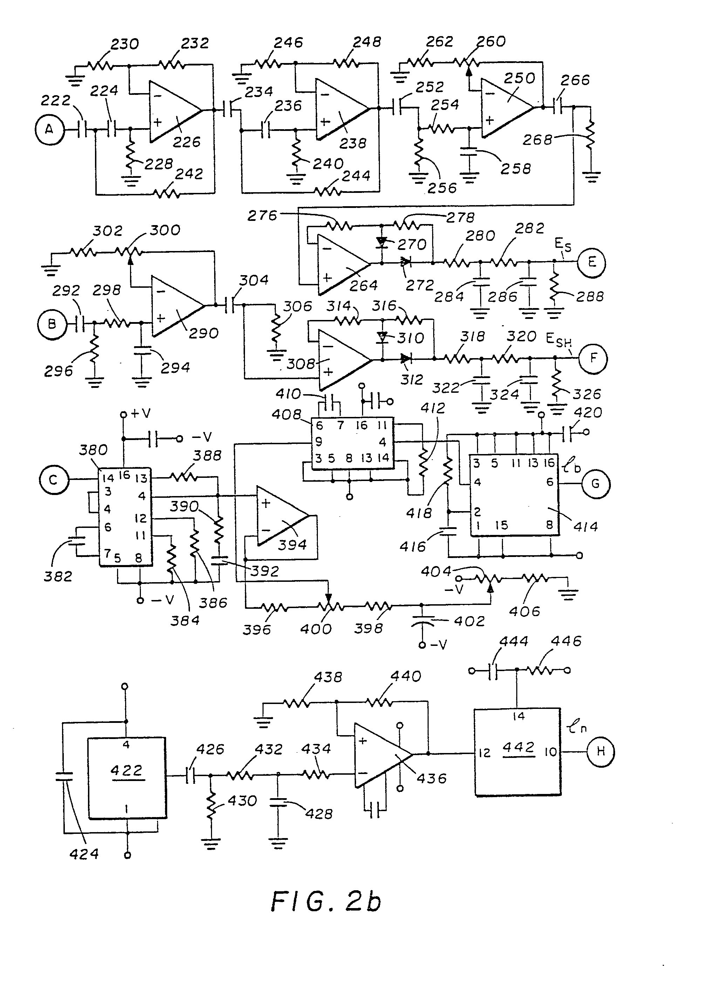

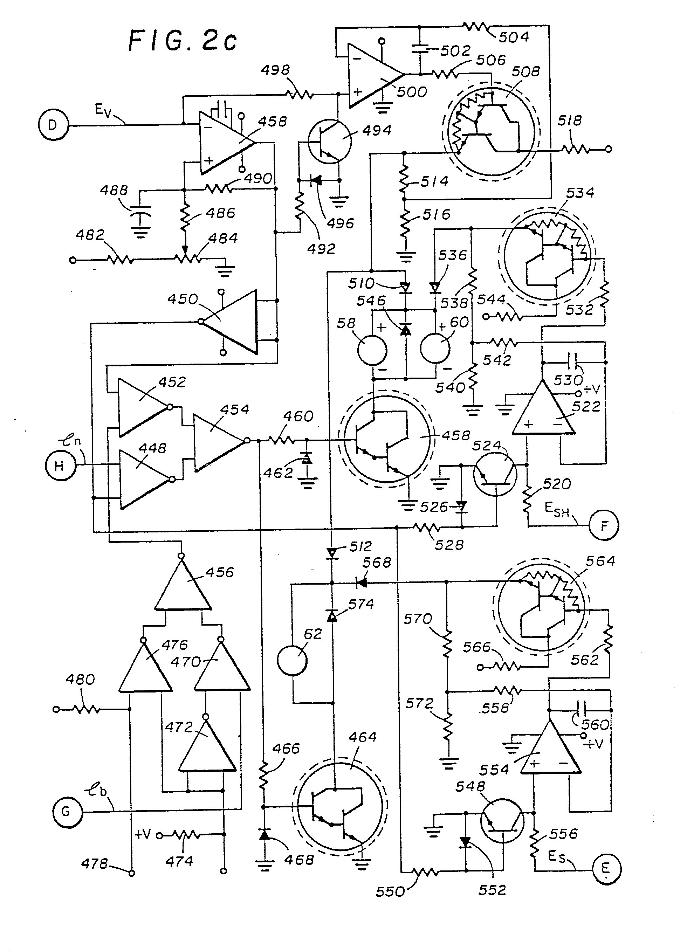

Referring to Figure 2, and in particular to Figure 2a, there is shown a detailed schematic of one embodiment of the present invention wherein the microphone 10, which may be a ECM-16 from the Sony Corporation, is connected to an input jack 64 connected by means of a shielded cable 66 through a coupling capacitor 68 to an input circuit of a differential amplifier 70. The input circuit of the amplifier 70 includes resistors 72 and 74 and grounded diodes 76 and 78. Connected to the inverting input of the amplifier 70 is a feedback network consisting of a resistor 80 in parallel with a capacitor 82. The level of the voltage at the inverting terminal of the amplifier 70 is established by a resistor 84. Supply voltages for the amplifier 70 are provided at terminals 86 and 88 connected to capacitors 90 and 92, respectively.

As connected, the amplifier 70 is the preamplifier 12 of Figure 1 and provides an output applied through a coupling capacitor 94 and through resistor 96 to terminal 6 of a compressor network 98 as part of the signal compressor 14. Typically, the compressor network 98 may be one half of an integrated circuit identified by part no. NE570N. Terminals referred to in this description will be with reference to examples of the integrated circuits that are referenced. A voltage supply is connected to the compressor network 98 directly to terminal 4 and through a capacitor 100 to terminal 1. Terminal 1 also connects to a resistor 102 which has one end grounded. Connected to terminal 5 of the compressor network 98 is a bias network consisting of resistors 104 and 106 interconnected to a capacitor 108. Also connected to the resistor 104 is a coupling capacitor 110, terminal 7 of the compressor network 98 and capacitors 112 and 114 connected, respectively, to terminals 2 and 3.

A compressed signal at the output of the network 98 is applied through the coupling capacitor 110 to the input of a differential amplifier 116 through an input resistor 118. Amplifier 116 forms part of the preamplifier 16 of Figure 1 that includes an input circuit to the inverting terminal consisting of a resistor 120 in series with a capacitor 122. Also connected to the inverting terminal is a feedback network consisting of a resistor 124 in parallel with a capacitor 126. The noninverting terminal of the amplifier 116 connects to a resistor 128 that has a second terminal grounded.

The output of the amplifier 116 is input to each of the channels of the filter array 18 with the output of the amplifier connected through filter capacitors 130 and 132 to the input of an amplifier 134 as a part of the high pass filter 24a of the low pass channel 24. Tied to the interconnection of the capacitors 130 and 132 is a filter resistor 136 with a resistor 138 connected to ground and to the input terminal of the amplifier. Connected to the inverting terminal of the amplifier 134 is a resistor 140 and a resistor 142, the latter as part of the feedback network.

An output of the amplifier 134 is applied through filter capacitors 144 and 146 to the input of an amplifier 148 that also forms a part of the high pass filter 24a of the low pass channel 24. Tied to the interconnection of the capacitors 144 and 146 is a filter resistor 150 and connected to the second terminal of the capacitor 146 is a resistor 152. The inverting terminal of the amplifier 148 connects to a resistor 154 and a resistor 156, the latter as a part of a feedback network.

At the output of the amplifier 148 there is generated the output of the high pass filter 24a which applied to the low pass filter 24b of Figure 1. The interconnection between the output of the amplifier 148 and the low pass filter 24b is by means of a filter circuit for a differential amplifier 158. Included as part of the filter circuit for the amplifier 158 are resistors 160 through 163 and a filter capacitor 164. The interconnection of the resistors 162 and 163 is tied to a filter capacitor 166 which also connects to the output of the amplifier 158. The inverting input terminal of the amplifier 158 is connected to resistors 168 and 170, the latter forming a feedback network for the amplifier.

The output of the amplifier 158 connects to the input circuit of an amplifier 172 where the input circuit consists of filter resistors 174 and 176, and a filter capacitor 178. Tied to the interconnection of the resistors 174 and 176 is a filter capacitor 180 that also connects to the output of the amplifier 172. A feedback network for the amplifier 172 includes a resistor 182 which is tied to the inverting input terminal of the amplifier and to a resistor 184. The output of the amplifier 172 is the output signal of the low pass channel 24 connected to the precision rectifier 28 and the comparator 26.

Also connected to the output of the amplifier 116 is a variable resistor 186 having a wiper arm tied to the input resistor 188 of an amplifier 190 as a part of the midfrequency channel 22. Connected to the input of the amplifier 190 at the resistor 188 is a feedback resistor 192 and the output of differential amplifiers 194 and 196, all a part of the midfrequency channel 22. Connected to the noninverting input terminal of the amplifier 190 is a resistor 198.

The output of the amplifier 190 is connected through a resistor 200 to an amplifier 202 that has a second input connected to a resistor 204 and a feedback network consisting of a capacitor 206. The output of the amplifier 202 is connected through a resistor 208 to one input of the amplifier 194 that has a second input connected to a resistor 210. A feedback capacitor 212 is connected across the amplifier 194. Also connected to the output of the amplifier 202 is a resistor 214 as part of an input circuit to the amplifier 196 that includes a second terminal connected to a resistor 216. A feedback network for the amplifier 196 consists of a resistor 218. The output of the amplifier 196 is connected through a resistor 220 to the input of the amplifier 190, as explained.

Referring to Figure 2b, the wiper arm of the variable resistor 186 is also connected to the input of the high pass channel 20. Specifically the wiper arm of the resistor 186 is tied through filter capacitors 222 and 224 to one input of an amplifier 226. The interconnection of the capacitors 222 and 224 is tied to a filter resistor 242 having a second terminal tied to the output of amplifier 226. Also connected to the capacitor 224 at the input of the amplifier 226 is an input resistor 228. The inverting input terminal of the amplifier 226 connects to resistors 230 and 232, the latter forming the feedback network for the amplifier.

The output of the amplifier 226 interconnects through filter capacitors 234 and 236 to one input of a differential amplifier 238 which along with the amplifier 226 forms the high pass filter of the channel 20. Also connected to the input of the amplifier 238 is an input resistor 240 with a filter resistor 244 tied to the interconnection of the capacitors 234 and 236. Tied to the inverting terminal of the amplifier 238 are resistors 246 and 248, the latter also connected to the output of the amplifier. At the output of the amplifier 238, there is generated the output signal of the high pass filter 20 connected to the precision rectifier 46.

The precision rectifier 46 includes an amplifier 250 connected to the output of the amplifier 238 through a coupling capacitor 252 and an input resistor 254. Forming a part of the input circuit to the amplifier 250 is a resistor 256 and a capacitor 258. The gain in the amplifier 250 is adjustable by means of a variable resistor 260 having a wiper arm connected to the inverting terminal of the amplifier and in series with a resistor 262. Also included as a part of the precision rectifier is an amplifier 264 connected to the output of the amplifier 250 through a coupling capacitor 266 having an interconnection to a resistor 268. The output of amplifier 264 is tied to the interconnection of diodes 270 and 274 with the former connected to a feedback resistor 276 and a resistor 278 which also connects to the diode 272. The interconnection of the diode 272 and the resistor 278 is tied to a filter consisting of resistors 280 and 282 and capacitors 284 and 286. The output of the precision rectifier 46 is generated across an output resistor 288 on the output line $E_s$.

Receiving the output of the midfrequency channel 22, generated at the output of the amplifier 202, is an amplifier 290 as part of the precision rectifier 36. The input circuit for the amplifier 290 includes capacitors 292 and 294 and resistors 296 and 298. Connected to the inverting terminal of the amplifier 290 is a gain adjustment variable resistor 300 in series with a resistor 302 with the wiper arm of the variable resistor connected to the terminal of the amplifier. The output signal from the amplifier 290 is coupled through a capacitor 304 to a resistor 306 as part of an input circuit for an amplifier 308 that is a part of the precision rectifier 36. Connected to the output of the amplifier 308 are diodes 310 and 312 with the diode 310 connected to resistors 314 and 316 and the diode 312 connected to resistor 316 and a filter network. Included in the filter network are resistors 318 and 320 along with capacitors 322 and 324. An output of the precision rectifier 36 is generated across a resistor 325 and appears on an output line $E_{SH}$.

Referring again to Figure 2a, mechanically interconnected to the variable resistor 186 by means of a linkage 328 is the wiper arm of a variable resistor 330 connected to the output of amplifier 172 as a part of the low pass channel 24. This wiper arm is interconnected by means of an input circuit to one terminal of an amplifier 332 as part of the precision rectifier 28. The input circuit consists of capacitors 334 and 336 along with resistors 338 and 340. The gain of the amplifier 332 is adjustable by means of a variable resistor 338 in series with a resistor 340 and connected to the output of the amplifier.

A voltage generated at the output terminal of the amplifier 332 is coupled through a capacitor 342 to the input of an amplifier 344 across a resistor 346. The output of the amplifier 344 is tied to diodes 348 and 350 with the former connected to resistors 352 and 354 where the diode 350 is also connected to the resistor 354 and to an output filter. The output filter consists of resistors 356 and 358 along with capacitors 360 and 362. The output of the precision rectifier 28 is generated across an output resistor 364 and appears on an output line $E_V$.

Also connected to the output of the amplifier 172 is a differential amplifier 366 as part of the comparator 26. The output of the amplifier 172 is connected to the input of the amplifier 366 through capacitors 368 and 370 with the input signal to the amplifier generated across the resistor 372 and connected to the amplifier through a resistor 374. The noninverting terminal of the amplifier 366 is tied to a resistor 376 and the amplifier includes a feedback network consisting of a resistor 378.

Returning to Figure 2b, an output voltage from the amplifier 366 is connected to the phase locked loop 30 including an integrated circuit 380 which typically may be a type CD4046. Terminal numbers illustrated in the integrated circuit 380 are identified with the type CD4046 with the output of the amplifier 366 connected to terminal 14. Terminals 6 and 7 of the circuit 380 are interconnected by means of a capacitor 382 with a voltage supply connected to terminals 5 and 8. Terminal 11 is connected to the voltage supply through a resistor 384 and terminal 12 is connected through a resistor 386 also to the voltage supply. The output of the circuit 380 is generated at the terminal 9 with a resistor 388 interconnecting terminal 9 to terminal 13. Terminal 9 also connects to a resistor 390 in series with a capacitor 392 to the voltage supply at terminal 8.

The output of the integrated circuit 380 at terminal 9 connects to the noninverting terminal of an amplifier 394 that has an inverting terminal connected directly to the output of the amplifier. The output of the amplifier 394 connects to a scaling circuit including resistors 396 and 398 in a series with a variable resistor 400. A voltage supply is connected through a capacitor 402 to the resistor 398 and also connects to an offset adjustment including a variable resistor 404 in series with a resistor 406 which is grounded.

Tied to the wiper arm of the variable resistor 400 is terminal 9 of an integrated circuit 408 that also may be a type CD4046 with the terminal numbers illustrated identified with this integrated circuit. Terminals 6 and 7 of the integrated circuit 408 are interconnected by a capacitor 410 and terminals 3, 5, 8, 13 and 14 are interconnected to a voltage supply. Terminal 11 is also connected to the voltage supply through a resistor 412. An output of the integrated circuit 408 is generated at terminal 4 and applied to terminal 4 of an integrated circuit 414 which typically may be a type MC14528. Terminals 1, 8 and 15 of the circuit 414 are interconnected to a voltage supply which also connects to terminal 2 through a capacitor 416. Terminals 2 and 3 of the circuit 414 are interconnected by means of a resistor 418 where terminal 3 is also interconnected with terminals 5, 11, 13 and 16 to a capacitor 420 which is connected to a voltage supply. The output signal of the circuit 414 in the form of pulses representing a frequency related to the formant vowel sounds is generated at terminal 6 and connects to the gating logic 48 and 50.

Illustrated in the lower portion of Figure 2b is the random frequency generator including an integrated circuit 422 with terminals 1 and 4 interconnected by means of a by-pass capacitor 424. Typically, the integrated circuit 422 is a type MM5837 integrated circuit from National Semi-Conductor. The output of the integrated circuit 422 is applied to the band pass filter 42 that consists of capacitors 426 and 428 along with resistors 430 and 432. The output of the filter is applied through a resistor 434 to the inverting terminal of a comparator amplifier 426. The noninverting terminal of the amplifier 436 is interconnected to a resistor 438 and a resistor 440, the latter connected in a feedback circuit that is tied to the output of the amplifier. Also tied to the output of the amplifier 436 is a one shot multivibrator which may be a type MC14528 integrated circuit. Terminal 12 of the integrated circuit 442 connects to the output of the amplifier 436 and terminal 413 connects to a timing network including a capacitor 444 in series with a resistor 446 with the capacitor connected to a negative voltage supply and the resistor connected to a positive voltage supply. The random frequency signal, that is amplitude modulated as explained previously, is generated at terminal 10 of the integrated circuit 442 and is connected to an input of a NOR gate 448, as illustrated in Figure 2c.

Referring to Figure 2c, the second input to the gate 448 is the output of an inverter 450 as part of the comparator 52. The gate 448 is part of the gating logic 48 that also includes gates 452 and 454. Input signals applied to the gate 452 include the output of a gate 456 and the output of a differential amplifier 458 as part of the comparator 52 where the output of the amplifier 458 also connects to the inputs of the inverter 450.

Input terminals to the gate 454 are connected to terminals of the gates 448 and 452. The output of the gate 454 is connected to an input circuit for a Darlington amplifier 458 where the input circuit consists of a resistor 460 and a diode 462. Also connected to the output of the gate 454 is a Darlington amplifier 464 by means of an input circuit consisting of a resistor 466 in series with a diode 468.

Connected to the output of the integrated circuit 414 of Figure 2b is one input of a gate 470 having an output tied to one input of the gate 456. A second input to the gate 470 is the output of an inverter 472 having inputs interconnected to a voltage supply and a resistor 474. Also connected to the resistor 474 is an input terminal of a gate 476 having a second input connected to a pitch signal at a terminal 478 and to a resistor 480.

As illustrated in Figure 1, the gating logic 48 and 50 comprises separate units while the schematic of Figure 2c shows the logic of these two units combined to include the gates 448, 452, 454, 456, 470 and 476 along with the inverters 450 and 472. Thus, the implementation of the circuit of Figure 1 combines the gating logic 48 and 50.

The output signal of the low pass filter on the line $E_V$ is applied to the inverting input terminal of the amplifier 458. The noninverting terminal is tied to an input circuit consisting of a resistor 482 in series with a variable resistor 484 that has a wiper arm tied to a resistor 486. The resistor 486 is interconnected to a capacitor 488 and to a resistor 490 as part of a feedback network for the amplifier 458.

As explained, the output of the amplifier 458 is tied to both inputs of the inverter 450 and to one input of the gate 452. Also connected to the output of the amplifier 458 through a resistor 492 is a transistor 494 having the base-emitter electrodes interconnected by means of a diode 496. The collector of the clamp transistor 494 is interconnected to a resistor 498 and is also tied to the noninverting terminal of amplifier 500 that forms a part of the power driver 54. The output of the amplifier 500 is interconnected through a capacitor 502 to the noninverting terminal which is also connected to a resistor 504. The output of the amplifier 500 is connected through a resistor 506 to a Darlington amplifier 508 which has an output as one drive voltage of the power driver 54 for the transducers 58 and 60. The output of the amplifier 508 is connected to the transducers 58 and 60 through a diode 510.

Since the output of the amplifier 508 is a signal controlled by the low pass channel 24, which as explained is applied to all three transducers, the output of the amplifier 508 is also connected to the transducer 62. This connection is through a diode 512.

Also included as part of the circuitry for the Darlington amplifier 508 is a resistance network including resistor 514 in series with a resistor 516. The interconnected collector electrodes of the Darlington pair are tied to a supply resistor 518.

Driving the transducers 58 and 60, in addition to the amplifier 508, is the output of the midfrequency channel 22 through the precision rectifier 36 which appears on the line $E_{SH}$, as illustrated in Figure 2b. This signal is applied through a resistor 520 to the input of an amplifier 522 that is also connected to the collector electrode of a transistor 524. The emitter and base electrodes of the transistor 524 are interconnected by means of a diode 526 with the base electrode connected to the output of the inverter 450 through a resistor 528. Connected to the inverting input terminal of the amplifier 522 is a capacitor 530 which also connects through a resistor 542 to the output of a Darlington amplifier output divider.

The amplifier 522, transistor 524, and the Darlington amplifier 534 are part of the power driver 54 connected to the transducers 58 and 60. As such, the output of the Darlington amplifier 534 is connected to the transducers 58 and 60 through a diode 536. The output of the amplifier 534 is also connected to a resistance network consisting of resistors 538 and 540. The interconnection of the resistors 538 and 540 is tied to a resistor 542, where the latter is connected to the inverting input of the amplifier 522. The interconnected collector electrodes of the amplifier 534 are tied to a voltage supply through a resistor 544.

Also driving the transducers 58 and 60 is the output of the gate 454 through the resistor 460 as connected to the Darlington amplifier 458. The amplifier 458 functions as a switch with the interconnected collector electrodes tied to the transducers 58 and 60 and the emitter electrode of the second transistor of the Darlington pair connected to ground. In parallel with the transducers 58 and 60 is a diode 546.

Thus, the transducers 58 and 60 are driven by the output of the low frequency channel 24 by means of the Darlington amplifier 508. In addition, the vibrators 58 and 60 are driven by the output of midfrequency channel 22 by means of the Darlington amplifier 534. However, before either of these amplifiers 508 and 534 will energize the transducers 58 and 60, the Darlington amplifier 458 must be conducting as controlled by the output of the gate 454. The gate 454 is controlled by the random frequency signal from the one shot multivibrator 38 and the low frequency formant vowel sounds as represented by the output of the voltage controlled oscillator 34.

As previously explained, the output of the low frequency channel 24 also drives the transducer 62. To drive the transducer 62 from the output of the low frequency channel 24 the output of the inverter 450 is connected to the base electrode of a transistor 548 through a base drive resistor 550. The emitter and base electrodes of the transistor 548 are interconnected by means of a diode 552 and the collector electrode is tied to one input of a differential amplifier 554. The noninverting terminal of the amplifier 554 is connected through a resistor 556 to the output of the precision rectifier 46 as appearing on the line $E_S$. The output of the amplifier 554 is interconnected to a resistor 562 and through a capacitor 560 to a resistor 558 and the inverting terminal of the amplifier. The resistor 562 connects to the input of a Darlington amplifier 564 having interconnected collector electrodes tied to a voltage supply through a resistor 566. The output of the Darlington amplifier 564 is applied through a diode 568 to the transducer 62 and also to a resistance network consisting of resistors 570 and 572. Connected in parallel with the transducer 62 is a diode 574 which is tied to the interconnected collector electrodes of the Darlington amplifier 464 along with the transducer 62.

Thus, as interconnected in Figure 2c, the transducer 62 is driven by the output of the low frequency channel 24 directly from the output of the Darlington amplifier 508 through the Darlington amplifier 464. The transducer 62 is also driven by the output of the high frequency channel 20 from the Darlington amplifier 564. Again, through the Darlington amplifier 464. The Darlington amplifier 464 is controlled by the output of the gate 454 which, as explained responds to the random noise frequency signal from the one shot multivibrator 38 and the low frequency signal at the output of the voltage controlled oscillator 34.

With the interconnection of components as illustrated in Figures 2a, 2b and 2c, the transducers 58, 60 and 62 produce sensations that are: (1) periodic, low frequency vibrations spread over a large surface, (2) aperiodic, high frequency stimulation (high frequency for the vibro-tactile-sense) spread over a large surface, and (3) aperiodic, high frequency stimulation over a small surface area, by means of the center vibrator 62. In use, the transducers are placed on the abdomen of a person having a hearing impairment to provide sensations by means of vibration to convey to the user of the tactile aid of the present invention perceptions to speech sound as coming from a single point.

Although only one embodiment of the invention is illustrated in the accompanying drawings and described in the foregoing detailed description, it will be understood that the invention is not limited to the embodiment disclosed, but is capable of rearrangements, modifications and substitutions without departing from the invention as defined in the accompanying claims.

**Claims**

1. A tactile aid for persons having a hearing impairment including a filter array (18) responsive to an acoustic signal and having a high pass channel (20), a low pass channel (24) and a midfrequency channel (22) each providing an output frequency, means for modulating (50) the amplitude of a random frequency signal by the output frequency of the high pass channel (20) to generate a high pass modulated signal, means for modulating (48) the amplitude of the random frequency signal by the output of the midfrequency channel (22) to generate a midfrequency signal, and vibrating means (54, 56, 58, 60, 62) for transducing the modulated signals, characterized by:

— first vibrating means (56, 62) responsive to the high pass modulated signal and the output frequency of the low pass channel (24) to produce a vibrating motion, and

— second vibrating means (54, 58, 60) responsive to the midfrequency modulated signal and the output frequency of the low pass channel (24) to produce a second vibrating motion.

2. A tactile aid as set forth in Claim 1, wherein the second vibrating means (54, 58, 60) is further characterized by a voltage controlled power driver (54) responsive to the midfrequency modulated signal and the output frequency of the low pass channel (54) for driving first and second vibrators (58, 60) and said first vibrating means (56, 62) is further characterized by a voltage controlled power driver (56) responsive to the high pass modulated signal and the output frequency of the low pass channel (24) to drive a third vibrator (62).

3. A tactile aid as set forth in Claim 1 further characterized by means (26, 30, 34) responsive to the output of the low pass channel (24) for detecting the first formant of vowel sounds and for converting the sounds into a low frequency signal applied to said first and second vibrating means (56, 62; 54, 58, 60).

4. A tactile aid as set forth in Claim 1 further characterized by first and second vibrators (58, 60) responsive to the midfrequency driver signal and each producing a vibrating motion in response thereto and a third vibrator (62) responsive to the high pass driver signal and producing a vibrating motion in response thereto.

5. A tactile aid as set forth in Claim 4 further characterized by gating means (48) for modulating a random frequency signal by the output of the high pass channel (20) to generate a high pass modulated signal and gating means (50) for modulating the random frequency signal by the midfrequency channel (22) to generate a midfrequency modulated signal.

**Patentansprüche**

1. Tasthilfe für Personen, die einen Hörschaden haben, mit einer Filteranordnung (18), die auf ein akustisches Signal anspricht und einen Hochpaßkanal (20), einen Tiefpaßkanal (24) und einen Mittelfrequenzkanal (22) aufweist, von denen jeder eine Ausgangsfrequenz liefert, mit Mitteln zum Modulieren (50) der Amplitude eines Zufallsfrequenzsignals durch die Ausgangsfrequenz des Hochpaßkanals (20), um ein moduliertes Hochpaßsignal zu erzeugen, mit Mitteln zum Modulieren (48) der Amplitude des Zufallsfrequenzsignals durch die Ausgabe des Mittelfrequenzkanals (22) für die Erzeugung eines Mittelfrequenzsignals, und mit Schwingungsmitteln (54, 56, 58, 60, 62) zum Umwandeln der modulierten Signale, gekennzeichnet durch:

— erste Schwingungsmittel (56, 62), die auf das modulierte Hochpaßsignal und die Ausgangsfrequenz des Tiefpaßkanals (24) ansprechen, um eine Schwingungsbewegung zu erzeugen, und

—zweite Schwingungsmittel (54, 58, 60), die auf das modulierte Mittelfrequenzsignal und die Ausgangsfrequenz des Tiefpaßkanals (24) ansprechen, um eine zweite Schwingungsbewegung zu erzeugen.

2. Tasthilfe nach Anspruch 1, dadurch gekennzeichnet, daß das zweite Schwingungsmittel (54, 58, 60) einen spannungsgesteuerten Leistungstreiber (54) aufweist, der von dem modulierten Mittelfrequenzsignal und der Ausgangsfrequenz des Tiefpaßkanals (54) angesteuert wird, um erste und zweite Vibratoren (58, 60) zu treiben und daß das erste Schwingungsmittel (56, 62) ferner einen spannungsgesteuerten Leistungstreiber (56) aufweist, der von dem modulierten Hochpaßsignal und der Ausgangsfrequenz des Tiefpaßkanals (24) angesteuert wird, um einen dritten Vibrator (62) zu treiben.

3. Tasthilfe nach Anspruch 1, gekennzeichnet durch Mittel (26, 30, 34), die auf die Ausgabe des Tiefpaßkanals (24) ansprechen und zur Erkennung des ersten Formanden von Vokaltönen und zur Umwandlung der Töne in ein niederfrequentes Signal dienen, das an die ersten und zweiten Schwingungsmittel (56, 62; 54, 58, 60) gelegt wird.

4. Tasthilfe nach Anspruch 1, gekennzeichnet durch erste und zweite Vibratoren (58, 60), die von dem mittelfrequenten Treibersignal angesteuert werden und jeweils eine Schwingungsbewegung in Abhängigkeit davon erzeugen und daß ein dritter Vibrator (62) von dem Hochpaß-Treibersignal angesteuert wird und eine Schwingungsbewegung in Abhängigkeit davon erzeugt.

5. Tasthilfe nach Anspruch 4, gekennzeichnet durch eine Torschaltung (48) zum Modulieren eines zufallsfrequenten Signals durch die Ausgabe des Hochpaßkanals (70), um ein moduliertes Hochpaßsignal zu erzeugen, und durch eine Torschaltung (50) zum Modulieren des Zufallsfrequenzsignals durch den mittelfrequenten Kanal (22), um ein moduliertes Mittelfrequenzsignal zu erzeugen.

**Revendications**

1. Dispositif d'aide tactile pour des personnes souffrant d'une insuffisance auditive, comportant une série de filtres (18) réagissant à un signal acoustique et comprenant un canal passe-haut (20), un canal passe-bas (24) et un canal de fréquence moyenne (22), produisant chacun une fréquence de sortie, un dispositif de modulation (50) de l'amplitude d'un signal de fréquence aléatoire par la fréquence de sortie du canal passe-haut (20) pour produire un signal modulé passe-haut, un dispositif de modulation (48) de l'amplitude du signal de fréquence aléatoire par la sortie du canal de fréquence moyenne (22) pour produire un signal de fréquence moyenne et un dispositif vibratoire (54, 56, 58, 60, 62) pour convertir les signaux modulés, caractérisé en ce qu'il comporte un premier dispositif vibratoire (56, 62) réagissant au signal modulé passe-haut et à la fréquence de sortie du canal passe-bas (24) en produisant un mouvement vibratoire et un second dispositif vibratoire (54, 58, 60), réagissant au signal modulé de fréquence moyenne et à la fréquence de sortie du canal passe-bas (24) en produisant un second mouvement vibratoire.

2. Dispositif d'aide tactile selon la revendication 1, dans lequel le second dispositif vibratoire (54, 58, 60) est caractérisé en outre par un circuit d'attaque de puissance commandé par tension (54), réagissant au signal modulé de fréquence moyenne et à la fréquence de sortie du canal passe-bas (54) pour attaquer un premier et un second vibrateurs (58, 60), ledit premier dispositif vibratoire (56, 62) étant en outre caractérisé par un circuit d'attaque de puissance commandé par tension (56) réagissant au signal modulé passe-haut et à la fréquence de sortie du canal passe-bas (24) pour attaquer un troisième vibrateurs (62).

3. Dispositif d'aide tactile selon la revendication 1, caractérisé en outre par un dispositif (26, 30, 34) réagissant à la sortie du canal passe-bas (24) en détectant le premier formant de sons de voyelles et en convertissant les sons en un signal de basse fréquence appliqué auxdits premier et second dispositifs vibratoires (56, 62; 54, 58, 60).

4. Dispositif d'aide tactile selon la revendication 1, caractérisé en outre par un premier et un second vibrateurs (58, 60) réagissant au signal d'attaque de fréquence moyenne, et produisant chacun un mouvement vibratoire en réponse à ce signal, et un troisième vibrateurs (62) réagissant au signal d'attaque passe-haut et produisant un mouvement vibratoire en réponse à ce signal.

5. Dispositif d'aide tactile selon la revendication 4, caractérisé en outre par un circuit à portes (48) destiné à moduler un signal de fréquence aléatoire par la sortie du canal passe-haut (20) pour produire un signal modulé passe-haut et un circuit à portes (50) destiné à moduler le signal de fréquence aléatoire par le canal de fréquence moyenne (22) pour produire un signal modulé de fréquence moyenne.

## Fig. 1

FILTERS (18)

INPUT AMPLIFICATION &
FUNCTIONAL FILTERING

- 10
- 12
- 14 COMPRESSOR
- 16
- HIGH-PASS $F_1$ — 24
- 24a
- 24b — LOW-PASS
- 22 — BAND-PASS $F_{SH}$
- 20 — HIGH-PASS $F_S$

FILTER OUTPUT CONDITIONING

- 26 COMPARATOR
- 30 PHASE-LOCKED LOOP
- 32 SCALING & OFFSET
- 34 VCO — $\ell_b$
- PRECISION RECTIFIER $E_V$
- 28
- PRECISION RECTIFIER — 36 $E_{SH}$
- PRECISION RECTIFIER $E_S$
- 46
- 44 NOISE SOURCE
- 42 BAND-PASS
- 40 COMPARATOR
- 38 ONE-SHOT — $\ell_n$

OUTPUT SELECTION & POWER DRIVERS

- 52 COMPARATOR
- $\ell_n$
- $\ell_b$
- $E_V$
- 48 GATING
- $\ell_b \& \ell_n$
- $E_{SH}$
- VOLTAGE-CONTROLLED POWER DRIVER — 54
- VIB — 58
- SIDE VIBRATION (SIDE CHANNEL)
- VIB — 60
- 50 GATING
- $\ell_b \& \ell_n$
- $E_S$
- VOLTAGE-CONTROLLED POWER DRIVER — 56
- CENTER VIBRATION (CENTER CHANNEL)
- VIB — 62

Fig. 2a

FIG. 2b

# FIG. 2c

0 054 078

4